# EUROPEAN PATENT SPECIFICATION

(11) **EP 4 630 096 B1**
(45) Date of publication and mention of the grant of the patent: **16.09.2026**
(21) Application number: 23840772.0
(22) Date of filing: 06.12.2023
(51) Int. Cl.: A61M 37/00

(54) **TATTOO MACHINE**
TÄTOWIERMASCHINE
DERMOGRAPHE

(30) Priority: 06.12.2022 IT 202200025104
(43) Date of publication of application: 15.10.2025
(73) Proprietor: Laterza, Andrea, 30035 Mirano (VE) (IT); Raccanello, Pierantonio, 30036 Santa Maria di Sala (VE) (IT); Laterza, Luca, 30038 Spinea (VE) (IT)
(72) Inventor: Laterza, Andrea, 30035 Mirano (VE) (IT); Raccanello, Pierantonio, 30036 Santa Maria di Sala (VE) (IT); Laterza, Luca, 30038 Spinea (VE) (IT)
(74) Representative: Milan, Guglielmo
(86) International application number: PCT/IB2023/062315
(87) International publication number: WO 2024/121774

(56) References cited:
- EP-A1- 2 944 349
- WO-A1-2015/160370
- CN-A- 112 386 788
- US-B2- 11 173 293

## Description

### Technical field

The invention relates to a tattoo machine comprising a handle.

### Technological background

Tattoo machines typically comprise a stylus handle extending in a predominantly axial direction and carrying a needle unit, preferably motorized, to drive a tattoo needle in a reciprocating motion in the same axial direction. The handle is gripped precisely like a stylus by the tattooer to guide the needle along the path of the desired design so as to inject the ink or pigments required to form said design beneath the skin.

This (stylus) shape of the handle enables close control of the movements of the needle, but has the drawback of fatiguing the tattooer's wrist, therefore requiring frequent breaks to rest the joints and muscles involved. Over time, joint and muscle fatigue makes it more difficult to control the movement of the needle, which may affect the precision of the design and damage the skin being tattooed. Another drawback of the stylus handle is that it is clearly difficult to use for someone who has had one or more fingers amputated.

Tattoo machines with a stylus handle are represented in numerous prior art documents, including the following: DE20004163, EA030185, EP0374355, FR2825028, FR3113378, US4771660, US5279552, US5810862, US20050055042, WO2015156715, WO2018127249, US11173293, CN112386788, WO2015/160370, EP2944349 and WO2019096936.

All of the documents indicated disclose tattoo machines in which the axial dimension of the handle as defined above is predominant over the other dimensions, resulting precisely in this stylus shape.

Another drawback of known tattoo machines - see for example CN112386788, CN204864528, WO2015160370 or EP2944349 - relates to the fact that the size and inertia of the external drive that drives the needle and the transmission means that connect the needle to the external drive can hamper the movements of the tattooer.

### Description of the invention

The invention is described in the claims.

The problem at the heart of the present invention is to provide a tattoo machine that is structurally and functionally designed to at least partially obviate one or more of the stated drawbacks in the cited prior art that are typical of stylus-handle tattoo machines.

Another objective of the invention is to provide a tattoo machine that is particularly effective or comfortable to use.

Another objective of the invention is to provide a tattoo machine capable of improved adjustment.

A further objective of the invention is to make available to the art a tattoo machine that improves the known solutions in the context of a simple, rational and relatively cheap solution.

This problem is resolved and these objectives are achieved, at least in part, by the invention with, according to a first aspect, a tattoo machine whose handle is of the palmar type. In this context, palmar handle means a handle that does not have an axial dimension (preferably relating in particular to the drive axis of the needle) that is clearly predominant over the other dimensions, and that is typically intended to be gripped with or towards the palm of the tattooer's hand, unlike a stylus handle, which is characterized by a predominant axial dimension and is normally gripped only between the tattooer's thumb, index finger and middle finger, remaining at the side of the palm of the hand. The tattoo machine according to the invention further comprises a drive system contained in the handle.

The present invention can also have one or more of the following preferred features in addition to those mentioned above.

In a preferred example of the invention, the handle comprises a three-dimensional gripping surface. Preferably, the tattoo machine (or the handle) further comprises a needle moveable in a reciprocating motion in an axial direction. In embodiments, the dimension of the handle, and more specifically the dimension of the gripping surface, in an axial direction is not predominant over the dimensions in the directions orthogonal to the axial direction. In embodiments, the handle has a gripping surface in the form of a polyhedron with more than six faces, or at least a three-dimensional curved surface.

Preferably, the three-dimensional curved surface is chosen from a spheroid, an ovoid, and an ellipsoid, or at least a portion of the aforementioned geometric figures. This definition includes regular figures, i.e. a spherical surface, and combinations of such surfaces, i.e. a gripping surface that is partly spheroid and partly ellipsoid or ovoid. In a most preferred example, the gripping surface is spherical.

Advantageously, the handle is hollow. This means that other components of the tattoo machine can be seated at least partially inside the handle, as set out more clearly below.

In embodiments, the needle is carried moveably in a cartridge. The cartridge is preferably configured to guide the reciprocating motion of the needle in the axial direction. Advantageously, the position of the cartridge on the handle can be adjusted in the axial direction.

The tattoo machine preferably comprises means for adjusting the axial operating depth of the needle. In embodiments, the cartridge is seated removably in a needle unit or support that for example comprises a threaded portion on which a travel limiter is screwed in an axially adjustable position to adjust the operating depth of the needle. In particular, the travel limiter enables the axial position of the cartridge in relation to the needle unit or support to be adjusted so as to adjust the length of the portion of the needle projecting axially from the free end of the cartridge. Naturally, the threaded coupling between the needle unit and the travel limiter is a non-limiting example and may be replaced with other example means for adjusting the axial operating depth of the needle, as set out more clearly below.

In a preferred example, a cap forming part of the gripping surface of the handle is built into the travel limiter to enable the adjustment of said travel limiter.

The adjustment is preferably made in the axial direction, i.e. in the direction of the reciprocating motion of the needle.

This reciprocating motion is preferably obtained using a kinematic system that includes a drive system.

The tattoo machine therefore comprises a drive system contained in the handle, in particular to drive the needle in a reciprocating motion. In this context, the term "drive system" should preferably be understood as a synonym of "drive" or "motor". The drive system preferably includes an electric motor.

It will be appreciated that the palmar handle can define a larger cavity inside it compared to a handle having a predominant axial dimension. Consequently, the palmar handle makes it easier to seat the drive system inside the handle itself.

The drive system is seated (i.e. contained) in the handle since this arrangement, in addition to providing a significant advantage in terms of compactness, helps to improve the effectiveness of the tattoo machine. In particular, seating the drive system in the handle helps to reduce the distance between the centre of mass of the tattoo machine and the centre of rotation of the tattooer's palm. Reducing this distance significantly reduces the inertial resistance of the drive system on the movements of the tattooer. This improves the handling of the tattoo machine. Furthermore, seating the drive system in the handle enables the tattooer to grip the handle from different angles without limitations caused by the size of the drive itself.

Furthermore, seating the drive system inside the handle obviates the need for cables or other transmission means that connect the needle to an external drive. This makes the movements of the tattooer more free. The tattoo machine also preferably comprises at least one rechargeable battery arranged inside the handle and connected to the electric motor to drive the needle. This obviates the need to connect the tattoo machine to a power cable during use, thereby making the movements of the tattooer more free.

The tattoo machine is also preferably provided with a light source to illuminate the working area of the needle. In one example, said light source is ring-shaped and is positioned on the handle such as to at least partially surround the needle. In this manner, the working area is never obscured by the shadow of the tattoo machine.

As mentioned above, the threaded coupling between the needle unit and the travel limiter may be replaced (or combined) with other example means for adjusting the axial operating depth of the needle.

In embodiments, the tattoo machine comprises adjustment means configured to adjust the position of the drive system in the handle in the axial direction of the reciprocating motion of the needle. This makes it possible to adjust the operating depth of the needle using means for adjusting the axial position of the drive system.

In particular, the drive system comprises an electric motor, the position of which in the handle can be adjusted in the axial direction of the reciprocating motion of the needle.

The adjustment means preferably comprise a threaded pin that can be screwed/unscrewed to move the electric motor in the axial direction.

The threaded pin preferably extends parallel to the axial direction. The threaded pin also preferably projects from the handle away from the electric motor on the side opposite the needle.

Some embodiments also include means for supporting the electric motor that, when subjected to excessive pressure transmitted by the needle in the axial direction, enable the electric motor (and the needle therewith) to move axially backwards, thereby preventing the needle from causing injury to the skin. The support means may include known elastic and/or magnetic elements.

According to a second aspect, a tattoo machine preferably comprises a handle, a drive system seated in the handle, a needle connected kinematically to the drive system and carried thereby in a reciprocating motion in an axial direction, and adjustment means configured to adjust the position of the drive system in the handle in the axial direction of the reciprocating motion of the needle.

Naturally, the features described with reference to the first aspect of the invention are also applicable to the second aspect, and vice versa. In particular, to adjust the operating depth of the needle, the threaded coupling between the needle unit and the travel limiter may be replaced or combined with means for adjusting the axial position of the drive system. Further preferred features are also described in the attached dependent claims and the description below.

### Short description of the drawings

The features and advantages of the invention can be better understood from the detail description below of some of the preferred, non-exclusive embodiments thereof, illustrated with reference to the attached drawings, in which:
- Figure 1 is a cross section view of a tattoo machine according to one embodiment of the invention,

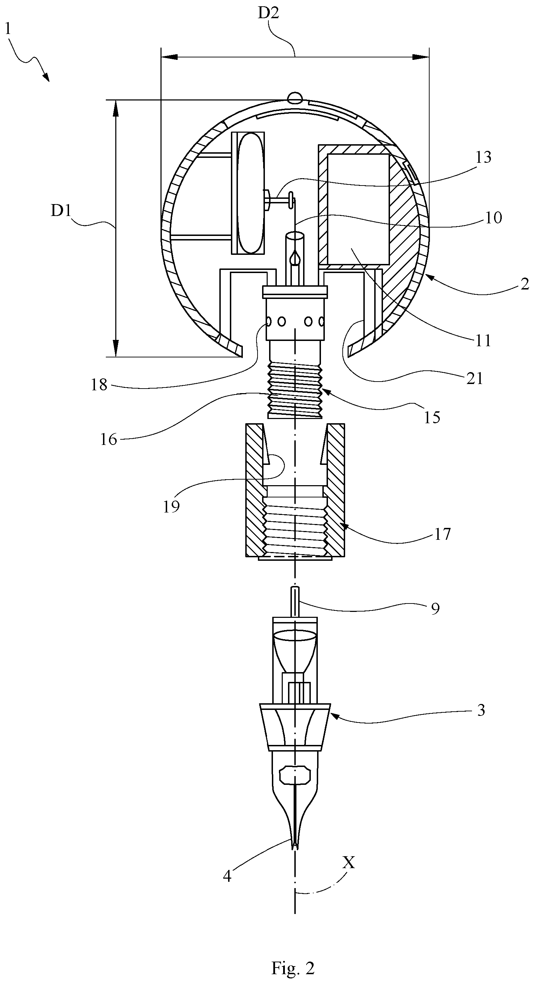

- Figure 2 is an exploded view of the tattoo machine in Figure 1,
- Figure 3 is a cross section view along the line III-III of a portion of the tattoo machine in Figure 1,
- Figure 4 shows a detail of the tattoo machine in Figure 1,
- Figure 5 is a cross section view of a tattoo machine according to another embodiment of the invention,
- Figure 6 is an exploded view of the tattoo machine in Figure 5,
- Figure 7 shows a detail of the tattoo machine in Figure 5,
- Figure 8 is a schematic cross section view of a tattoo machine according to another embodiment of the invention.

### Preferred embodiment of the invention

In the figures, equivalent details are usually indicated using the same reference numbers. The tattoo machine according to the present invention is denoted as a whole by reference number 1. The tattoo machine 1 comprises a handle 2, preferably hollow, that is used by the tattooer to grip the tattoo machine and to guide it over the surface of the skin of the patient according to a predetermined design to make a tattoo.

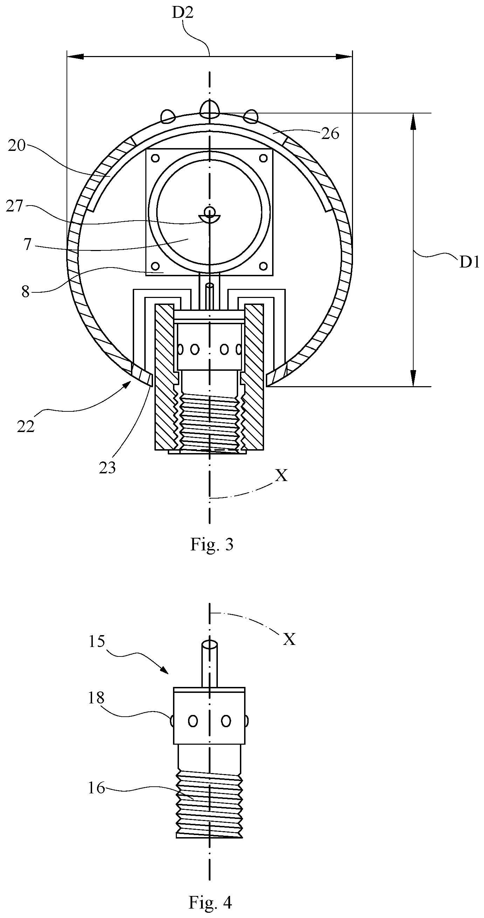

In embodiments, the design is made on the skin using a needle 4 capable of reciprocating motion (as set out more clearly below) in an axial direction X. It is therefore preferred that the tattoo machine 1 comprises a needle 4. The needle 4 is preferably carried moveably in a cartridge 3, which is in itself structurally known. The cartridge 3 is in turn preferably removably seated in a needle unit or support 15, shown for example in Figure 4. In embodiments, the needle unit or support 15 is also structurally known and is for example the type sold by the company Arklyn Project, in particular as a component of the "Metal Brush" tattoo machine model.

According to a first aspect of the invention, the handle 2 is of the palmar type, i.e. it preferably comprises a three-dimensional gripping surface 5 preferably formed such that the dimension D1 in the axial direction X is not predominant over the dimensions D2 in the directions orthogonal to the axial direction, which is however typical in stylus handles. Advantageously, the gripping surface 5 can therefore be arranged to be gripped by bearing against the palm of the tattooer's hand, and not necessarily clamped just between the thumb, index finger, and middle finger, although the surface 5 can obviously also be gripped with the aforementioned fingers or otherwise, according to the tattooer's habits. By way of example, surfaces that enable such an arrangement include polyhedral surfaces with more than six faces, or three-dimensional curved surfaces. Preferably, the three-dimensional curved surface is chosen from a spheroid, an ovoid, and an ellipsoid. The at least partially spherical surface is however preferred since it is most suited to being gripped in different ways and directions, as required.

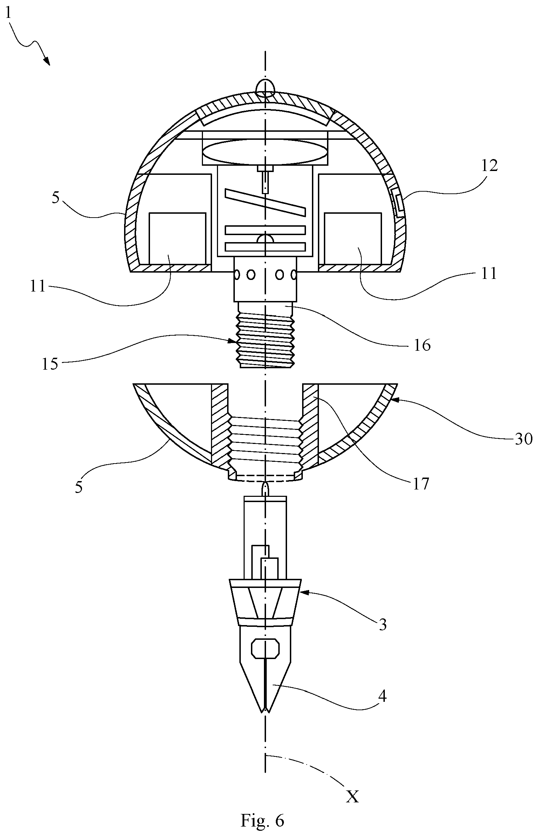

In embodiments, the needle 4 is moved in the direction of the axis X by a drive system 6, for example including an electric motor 7 mounted inside the handle 2 through a support 8 and connected to an appendage 9 of the needle 4 by a connecting rod 10. The motor 7, for example of the type sold by the company Faulhaber, is preferably powered by a rechargeable battery 11, which is also seated inside the handle 2. Recharging can be effected using a socket 12, preferably of the USB type. The battery 11 is preferably seated removably in the handle 2, so as to be easily replaceable when flat. Some embodiments, for example as shown in Figure 6, are provided with seats for two rechargeable batteries, both denoted with reference number 11. However, the motor 7 may be powered differently, for example using a connection outside the handle 2.

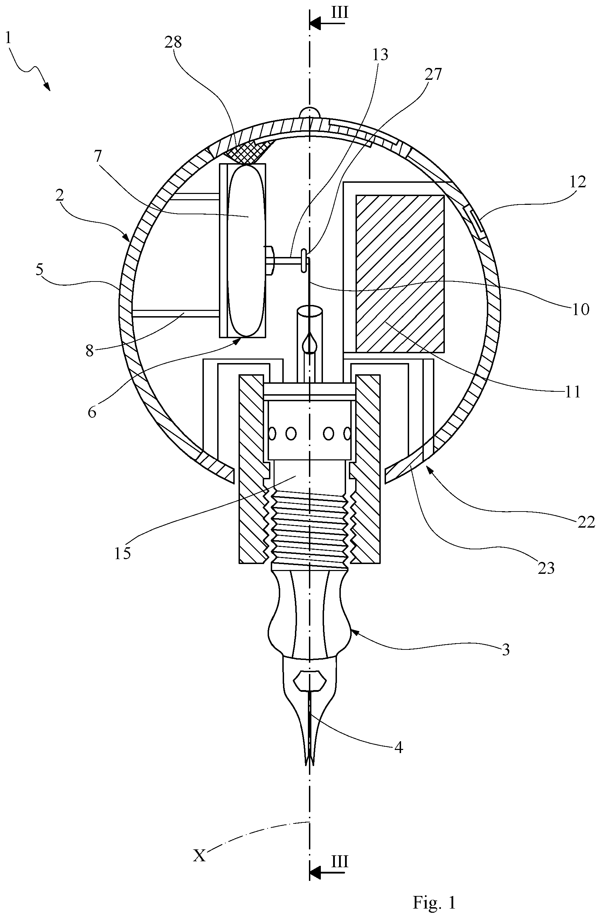

As shown for example in Figure 1, some embodiments include means 28 for supporting the electric motor 7 that, when subjected to excessive pressure transmitted by the needle 4 in the axial direction X, enable the electric motor (and the needle therewith) to move axially and reversibly backwards, thereby preventing the needle 4 from causing injury to the skin. The support means 28 may include known elastic and/or magnetic elements interposed between the electric motor 7 and the inner surface of the handle 2 on the side opposite the needle 4.

The needle unit or support 15 preferably comprises a threaded portion 16 on which a travel limiter 17 is screwed in an axially adjustable position. Screwing/unscrewing the travel limiter 17 along the threaded portion 16 enables the cartridge 3 to be moved axially, so as to adjust the length of the portion of the needle 4 projecting axially from the free end of the cartridge. Naturally, the threaded coupling between the support 15 and the travel limiter 17 can be replaced by other types of coupling or by any other suitable means enabling adjustment of the axial position of the cartridge 3.

In embodiments, a constraint system, for example comprising appendages 18 and teeth 19, prevents the relative rotation between the travel limiter 17 and the support 15 to maintain the adjustment setting. Additionally or alternatively, one or more O-rings may be fitted to the threaded portion 16 to ensure stability between the support 15 and the travel limiter 17.

In embodiments, the needle unit or support 15 is arranged in the region of a recess 21 in the handle 2. The recess 21 preferably extends in the axial direction X.

Advantageously, the support 15 is surrounded by a flange 22 that contains a light source 23, for example an LED light source. A circuit 20, preferably a PCB, for example rigid or flexible, is also arranged in the handle 2 to control the motor 7 and the light source 23 of the flange 22. The flange 22 is preferably made of a translucent material and acts as a light diffuser to illuminate the working area of the needle 4. In embodiments, the tattoo machine 1 also comprises a laser pointer aimed at the working area of the needle 4.

In embodiments, the tattoo machine 1 further comprises a display 26. The display 26 is preferably connected operationally to the circuit 20 and may be built into the handle 2 on the side opposite the needle 4.

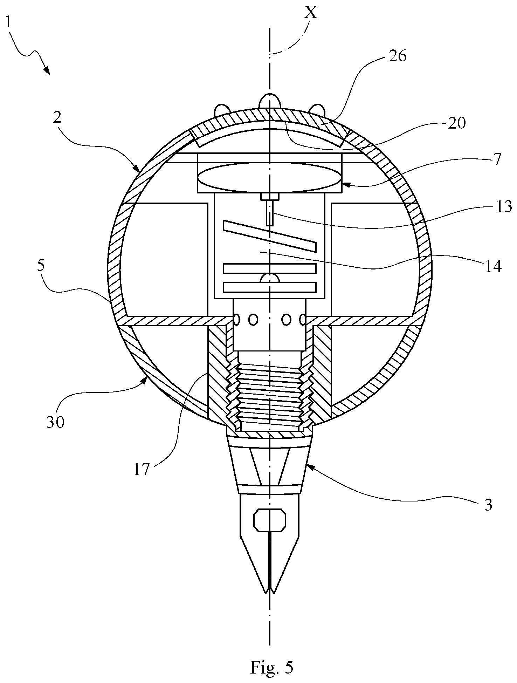

In a variant embodiment of the invention, shown in the example in Figure 5, the travel limiter 17 is built into a portion of the handle shaped in this instance like a cap 30. In addition to closing the bottom of the handle 2, the cap 30 is therefore easy to remove by unscrewing the travel limiter 17 from the threaded portion 16 of the support 15 and, once removed, provides easy access to the components of the tattoo machine inside the handle. Furthermore, this configuration helps to reduce size and in particular the axial projection of the needle on the side opposite the handle. In embodiments, the drive system 6 is of the rotary type. In particular, the electric motor 7 has a rotary shaft 13.

In the example in Figure 1, the axis of rotation of the shaft 13 is orthogonal to and incident with the axial direction X of the reciprocating motion of the needle. In this case, the rotary motion of the motor 7 can be transmitted to the needle 4 following conversion into a linear reciprocating motion through the connecting rod 10. For this purpose, the connecting rod 10 preferably comprises an eccentric element 27 fastened to the free end of the shaft 13.

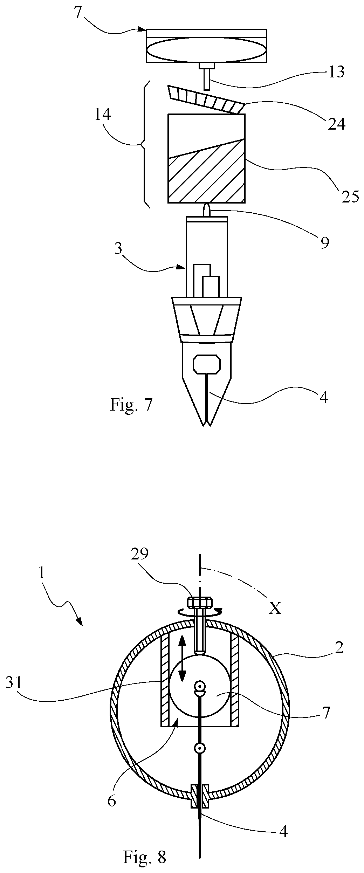

Alternatively, as shown in the example in Figure 5, the axis of rotation of the shaft 13 can be aligned with the axial direction X of the reciprocating motion of the needle. In this case, a different known transmission system 14 is preferably used to drive the needle. As shown in the example in Figure 7, the transmission system 14 for example comprises a cam 24 fastened to the free end of the shaft 13 and a cam follower 25 fastened to the appendage 9 of the needle. In embodiments, the cam 24 is formed by a swashplate, for example of the type known in pistons for axial pumps. According to a second aspect of the invention, which may possibly be combined with one or more of the features described in relation to the first aspect of the invention, although not necessarily, adjustment means 29 are provided to adjust the position of the drive system 6 in the handle 2 in the axial direction X of reciprocating motion of the needle, as shown in the example in Figure 8.

In particular, the position of the electric motor 7 in the handle 2 is adjustable in the axial direction X of the reciprocating motion of the needle. The adjustment means 29 preferably comprise a threaded pin that can be screwed/unscrewed to move the electric motor 7 in the axial direction X.

The threaded pin preferably extends parallel to the axial direction X. The threaded pin also preferably projects from the handle 2 away from the electric motor on the side opposite the needle 4.

Some embodiments further include a guide 31 configured to guide the electric motor 7 in translation in the axial direction X.

The tattoo machine according to the invention thus resolves the stated problem, achieving numerous advantages, including:
- improved ergonomics, enabling large and very detailed tattoos to be completed without joint fatigue in the arm and hand, thereby improving the precision of the tattooed design,
- advantageous illumination of the zone in which the tattoo is being made, thereby reducing the need for external lighting sources,
- easy adjustment of the operating depth of the needle by adjusting the travel limiter and/or the axial position of the drive system,
- easy access to the components of the tattoo machine arranged inside the handle.

## Claims

1. A tattoo machine (1) comprising a palmar-type handle (2) that does not have an axial dimension that is clearly predominant over the other dimensions, and that is typically intended to be gripped with or towards the palm of the tattooer's hand and a drive system (6) contained in said handle (2).

2. The tattoo machine (1) according to claim 1, wherein said drive system (6) includes an electric motor (7).

3. The tattoo machine (1) according to claim 1 or 2, wherein said drive system (6) is of the rotary type.

4. The tattoo machine (1) according to one or more of the preceding claims, comprising said drive system (6) contained in said handle (2) to drive a needle (4) in a reciprocating motion.

5. The tattoo machine (1) according to claim 4, wherein said handle (2) comprises a three-dimensional gripping surface (5) and said needle (4) is moveable in a reciprocating motion in an axial direction (X), wherein the dimension (D1) of said gripping surface (5) in the axial direction (X) is not predominant over the dimensions (D2) in the directions orthogonal to the axial direction (X).

6. The tattoo machine (1) according to claim 5, wherein said gripping surface (5) is chosen from a polyhedral surface with more than six faces and a three-dimensional curved surface.

7. The tattoo machine (1) according to claim 6, wherein said three-dimensional curved surface is chosen from a spheroid, an ovoid, and an ellipsoid.

8. The tattoo machine (1) according to one or more of the preceding claims when dependent on claim 4, wherein said needle (4) is carried moveably in a cartridge (3), the position of which on the handle (2) can be adjusted in the axial direction (X).

9. The tattoo machine (1) according to one or more of the preceding claims when dependent on claims 2 and 4, comprising at least one rechargeable battery (11) arranged in said handle (2) and connected to the electric motor (7) to drive the needle (4).

10. The tattoo machine (1) according to one or more of the preceding claims when dependent on claim 4, comprising a light source (23) configured to illuminate the working area of the needle (4).

11. The tattoo machine (1) according to claim 10, wherein said light source (23) is ring-shaped and arranged on the handle (2) such as to at least partially surround said needle (4).

12. The tattoo machine (1) according to one or more of the preceding claims when dependent on claim 8, wherein said cartridge (3) is seated removably in a support (15) that comprises a threaded portion (16) on which a travel limiter (17) is screwed in an axially adjustable position to adjust the operating depth of the needle (4).

13. The tattoo machine (1) according to claim 12, comprising a cap (30) forming part of the gripping surface (5) of the handle and built into said travel limiter (17) to enable the adjustment of said travel limiter.

14. The tattoo machine (1) according to one or more of the preceding claims when dependent on claim 4, comprising adjustment means (29) configured to adjust the position of the drive system (6) in the handle (2) in the axial direction (X) of the reciprocating motion of the needle (4).

## Patentansprüche

1. Tätowiermaschine (1) mit einem palmaren Griff (2), der keine axiale Abmessung aufweist, die deutlich gegenüber den anderen Abmessungen überwiegt, und der typischerweise dazu bestimmt ist, um mit oder in Richtung der Handfläche des Tätowierers ergriffen zu werden, und mit einem Antriebssystem (6), das im Griff (2) enthalten ist.

2. Tätowiermaschine (1) nach Anspruch 1, wobei das Antriebssystem (6) einen Elektromotor (7) umfasst.

3. Tätowiermaschine (1) nach Anspruch 1 oder 2, wobei das Antriebssystem (6) ein Rotationstyp ist.

4. Tätowiermaschine (1) nach einem oder mehreren der vorhergehenden Ansprüche, die das im Griff (2) enthaltene Antriebssystem (6) zum Antreiben einer Nadel (4) in einer Hin- und Herbewegung aufweist.

5. Tätowiermaschine (1) nach Anspruch 4, wobei der Griff (2) eine dreidimensionale Grifffläche (5) aufweist und die Nadel (4) in einer Hin- und Herbewegung in axialer Richtung (X) beweglich ist, wobei die Abmessung (D1) der Grifffläche (5) in axialer Richtung (X) gegenüber den Abmessungen (D2) in den Richtungen orthogonal zur axialen Richtung (X) nicht überwiegt.

6. Tätowiermaschine (1) nach Anspruch 5, wobei die Grifffläche (5) aus einer Polyederfläche mit mehr als sechs Flächen und einer dreidimensional gekrümmten Oberfläche ausgewählt wird.

7. Tätowiermaschine (1) nach Anspruch 6, wobei die dreidimensional gekrümmte Oberfläche aus einem Sphäroid, einem Ovoid und einem Ellipsoid ausgewählt wird.

8. Tätowiermaschine (1) nach einem oder mehreren der vorhergehenden Ansprüche, sofern von Anspruch 4 abhängig, wobei die Nadel (4) beweglich in einer Patrone (3) gelagert wird, deren Position am Griff (2) in axialer Richtung (X) eingestellt werden kann.

9. Tätowiermaschine (1) nach einem oder mehreren der vorhergehenden Ansprüche, sofern von Anspruch 2 und 4 abhängig, mit zumindest einer wiederaufladbaren Batterie (11), die im Griff (2) angeordnet und mit dem Elektromotor (7) verbunden ist, um die Nadel (4) anzutreiben.

10. Tätowiermaschine (1) nach einem oder mehreren der vorhergehenden Ansprüche, sofern von Anspruch 4 abhängig, mit einer Lichtquelle (23), die eingerichtet ist, um den Arbeitsbereich der Nadel (4) zu beleuchten.

11. Tätowiermaschine (1) nach Anspruch 10, wobei die Lichtquelle (23) ringförmig ist und am Griff (2) derart angeordnet ist, um die Nadel (4) zumindest teilweise zu umschließen.

12. Tätowiermaschine (1) nach einem oder mehreren der vorhergehenden Ansprüche, sofern von Anspruch 8 abhängig, wobei die Patrone (3) entfernbar in einer Halterung (15) sitzt, die einen Gewindeabschnitt (16) aufweist, auf den ein Hubbegrenzer (17) in einer axial verstellbaren Position aufgeschraubt ist, um die Arbeitstiefe der Nadel (4) einzustellen.

13. Tätowiermaschine (1) nach Anspruch 12 mit einer Kappe (30), die einen Teil der Grifffläche (5) des Griffs bildet und in den Hubbegrenzer (17) eingebaut ist, um die Einstellung des Hubbegrenzers zu ermöglichen.

14. Tätowiermaschine (1) nach einem oder mehreren der vorhergehenden Ansprüche, sofern von Anspruch 4 abhängig, mit einer Einstelleinrichtung (29), die eingerichtet ist, um die Position des Antriebssystems (6) im Griff (2) in axialer Richtung (X) der Hin- und Herbewegung der Nadel (4) einzustellen.

## Revendications

1. Machine à tatouer (1) comprenant une poignée de type palmaire (2) qui ne présente pas de dimension axiale nettement prédominante par rapport aux autres dimensions, et qui est typiquement destinée à être saisie avec ou vers la paume de la main du tatoueur, et un système d'entraînement (6) contenu dans ladite poignée (2).

2. Machine à tatouer (1) selon la revendication 1, dans laquelle ledit système d'entraînement (6) comprend un moteur électrique (7).

3. Machine à tatouer (1) selon la revendication 1 ou 2, dans laquelle ledit système d'entraînement (6) est de type rotatif.

4. Machine à tatouer (1) selon l'une ou plusieurs des revendications précédentes, comprenant ledit système d'entraînement (6) contenu dans ladite poignée (2) pour entraîner une aiguille (4) dans un mouvement alternatif.

5. Machine à tatouer (1) selon la revendication 4, dans laquelle ladite poignée (2) comprend une surface de préhension tridimensionnelle (5) et ladite aiguille (4) est mobile dans un mouvement alternatif dans une direction axiale (X), dans laquelle la dimension (D1) de ladite surface de préhension (5) dans la direction axiale (X) n'est pas prédominante par rapport aux dimensions (D2) dans les directions orthogonales à la direction axiale (X).

6. Machine à tatouer (1) selon la revendication 5, dans laquelle ladite surface de préhension (5) est choisie parmi une surface polyédrique à plus de six faces et une surface courbe tridimensionnelle.

7. Machine à tatouer (1) selon la revendication 6, dans laquelle ladite surface courbe tridimensionnelle est choisie parmi un sphéroïde, un ovoïde et un ellipsoïde.

8. Machine à tatouer (1) selon l'une ou plusieurs des revendications précédentes lorsqu'elle dépend de la revendication 4, dans laquelle ladite aiguille (4) est portée de manière mobile dans une cartouche (3) dont la position sur la poignée (2) peut être réglée dans la direction axiale (X).

9. Machine à tatouer (1) selon l'une ou plusieurs des revendications précédentes lorsqu'elle dépend des revendications 2 et 4, comprenant au moins une batterie rechargeable (11) disposée dans ladite poignée (2) et reliée au moteur électrique (7) pour entraîner l'aiguille (4).

10. Machine à tatouer (1) selon l'une ou plusieurs des revendications précédentes lorsqu'elle dépend de la revendication 4, comprenant une source lumineuse (23) configurée pour éclairer la zone de travail de l'aiguille (4).

11. Machine à tatouer (1) selon la revendication 10, dans laquelle ladite source lumineuse (23) est en forme d'anneau et disposée sur la poignée (2) de manière à entourer au moins partiellement ladite aiguille (4).

12. Machine à tatouer (1) selon l'une ou plusieurs des revendications précédentes lorsqu'elle dépend de la revendication 8, dans laquelle ladite cartouche (3) est logée de manière amovible dans un support (15) qui comprend une partie filetée (16) sur laquelle un limiteur de course (17) est vissé dans une position réglable axialement pour régler la profondeur de travail de l'aiguille (4).

13. Machine à tatouer (1) selon la revendication 12, comprenant un capuchon (30) faisant partie de la surface de préhension (5) de la poignée et intégré dans ledit limiteur de course (17) pour permettre le réglage dudit limiteur de course.

14. Machine à tatouer (1) selon l'une ou plusieurs des revendications précédentes lorsqu'elle dépend de la revendication 4, comprenant des moyens de réglage (29) configurés pour régler la position du système d'entraînement (6) dans la poignée (2) dans la direction axiale (X) du mouvement alternatif de l'aiguille (4).
